# EUROPEAN PATENT SPECIFICATION

(11) **EP 1 578 785 B1**
(45) Date of publication and mention of the grant of the patent: **13.02.2008**
(21) Application number: 03781116.3
(22) Date of filing: 29.12.2003
(51) Int. Cl.: C07K 7/06, C07K 7/08, A61K 47/48, C07K 14/475, C07K 14/71

(54) **PEPTIDES THAT BIND TO THE HEPARIN BINDING DOMAIN OF VEGF AND VEGFR-2**
PEPTIDE, DIE DIE HEPARINBINDUNGSDOMÄNE VON VEGF UND VEGFR-2 BINDEN
PEPTIDES SE LIANT AVEC LE DOMAINE DE LIAISON DE L'HEPARINE DE VEGF ET DE VEGFR-2

(30) Priority: 30.12.2002 NO 20026286
(43) Date of publication of application: 28.09.2005
(73) Proprietor: GE Healthcare AS, 0401 Oslo (NO)
(72) Inventor: KULSETH, Mari, Ann, N-0486 Oslo (NO)
(74) Representative: Hammett, Audrey Grace Campbell
(86) International application number: PCT/NO2003/000444
(87) International publication number: WO 2004/058802

(56) References cited:
- WO-A-01/52875
- WO-A-97/02479
- WO-A-99/10498
- WO-A-99/14321
- WO-A-99/40947
- WO-A-03/007689
- WO-A-03/074005
- US-A- 5 948 667
- US-A- 5 981 229
- US-A1- 2002 114 780
- DATABASE HCAPLUS [Online] MILLENNIUM PHARMACEUTICALS, INC.: "Humanized anti mammalian CC-chemokine receptor 2 (CCR2) antibodies and uses in therapeutics, prophylaxis and diagnosis" retrieved from STN INTERNATIONAL Database accession no. 2001:582061 XP002279014 -& WO 01 57226 A (MILLENIUM PHARMACEUTICALS, INC.) 9 August 2001 (2001-08-09)
- EL-SHEIKH AMR ET AL: "A novel vascular endothelial growth factor heparin-binding domain substructure binds to glycosaminoglycans in vivo and localizes to tumor microvascular endothelium." CANCER RESEARCH, vol. 62, no. 23, 1 December 2002 (2002-12-01), pages 7118-7123, XP002279013 ISSN: 0008-5472 (ISSN print)

## Description

### Field of invention

The present invention relates to peptide-based compounds and their use in therapeutically effective treatment as well as for diagnostic imaging techniques. More specifically the invention relates to the use of such peptide-based compounds as targeting agents that bind to the heparin binding domain of vascular endothelial growth factor and its receptor vascular endothelial growth factor receptor 2 (VEGFR2/KDR(kinase insert domain-containing receptor)/flk-1 (fetal liver kinase)). VEGFR-2 is expressed on angiogenic endothelial cells, haematopoietic stem cells, endothelial precursor cells in the bone marrow, and several malignant cells. Contrast agents based on these peptides may thus be used for diagnosis of for example malignant diseases, heart diseases, endometriosis, inflammation-related diseases and rheumatoid arthritis. Moreover such agents may be used in therapeutic treatment of these diseases through inhibition of angiogenesis.

### Background of invention

New blood vessels can be formed by two different mechanisms: angiogenesis or vasculogenesis. Angiogenesis is the formation of new blood vessels by sprouting/branching from existing vessels. The primary stimulus for this process may be inadequate supply of nutrients and oxygen (hypoxia) to cells in a tissue. The cells may respond by secreting angiogenic factors, of which there is many; one example, which is frequently referred to, is vascular endothelial growth factor (VEGF). These factors initiate the secretion of proteolytic enzymes that break down the proteins of the basement membrane, as well as inhibitors that limit the action of these potentially harmful enzymes. The other prominent effect of angiogenic factors is to cause endothelial cells to migrate and divide. Endothelial cells that are attached to the basement membrane, which forms a continuous sheet around blood vessels on the contralumenal side, do not undergo mitosis. The combined effect of loss of attachment and signals from the receptors for angiogenic factors is to cause the endothelial cells to move, multiply, and rearrange themselves, and finally to synthesise a basement membrane around the new vessels. Vasculogenesis is the generation of new vessels by recruiting endothelial precursor cells from the bone marrow. Newly published data shows that vasculogenesis not only is restricted to fetal blood vessel formation, but also occurs in the adult as response to various conditions. The bone marrow derived endothelial precursor cells recruited are also expressing VEGFR2.

Angiogenesis is prominent in the growth and remodelling of tissues, including wound healing and inflammatory processes. Tumours must initiate angiogenesis when they reach millimetre size in order to keep up their rate of growth. Angiogenesis is accompanied by characteristic changes in the endothelial cells and in their environment. The surface of these cells is remodelled in preparation for migration, and cryptic structures are exposed where the basement membrane is degraded, in addition to the variety of proteins which are involved in effecting and controlling proteolysis. In the case of tumours, the resulting network of blood vessels is usually disorganised, with the formation of sharp kinks and also arteriovenous shunts. Various glycosaminoglycans (GAGs) including heparan sulphate are important players in the angiogenic interactions. Several growth factors and their receptors including VEGF and VEGFR-2 have binding sites for heparan sulphate. Peptide mimics of GAGs may have important functions both as angiogenesis specific imaging agents, and as potential therapeutical agents through inhibition of angiogenesis.

Inhibition of angiogenesis is considered to be a promising strategy for anti tumour therapy. The transformations accompanying angiogenesis are also very promising as targets for diagnosis. An obvious example is malignant disease, but the concept also shows great promise in inflammation and a variety of inflammation-related diseases, including atherosclerosis. The macrophages of early atherosclerotic lesions are potential sources of angiogenic factors. These factors are also involved in re-vascularisation of infarcts In the myocardium.

Further examples of undesired conditions that are associated with neovascularization or angiogenesis implying the development or proliferation of new blood vessels, are shown below. Reference is also made in this regard to WO 98/47541.

Diseases and indications associated with angiogenesis are e.g. different forms of cancer and metastasis, e.g. breast, skin, colorectal, pancreatic, prostate, lung or ovarian cancer.

Other diseases and indications are inflammation (e.g. chronic), atherosclerosis, rheumatoid arthritis and gingivitis.

Further diseases and indications associated with angiogenesis are arteriovenous malformations, astrocytomas, choriocarcinomas, glioblastomas, gliomas, hemangiomas (childhood, capillary), hepatomas, hyperplastic endometrium, ischemic myocardium, Kaposi sarcoma, macular degeneration, melanoma, neuroblastomas, occluding peripheral artery disease, osteoarthritis, psoriasis, retinopathy (diabetic, proliferative), scleroderma, seminomas and ulcerative colitis.

The malignant cells and the stroma cells up-regulate proteins that are involved in the process of angiogenesis. More or less specific markers are expressed on the endothelial cells. These markers include growth factor receptors such as VEGFR2. Immunohistochemical studies in combination with electron microscopy have demonstrated that VEGFR2 is expressed on the abluminal and luminal plasma membranes of vascular endothelial cells (Dvorak & Feng, 2001 J Histochem Cytochem, 49:419). VEGF produced by hypoxic tumour cells or stromal cells binds to the VEGFR2 on endothelial cells and stimulate angiogenesis. As complexes of VEGF and VEGFR2 are found predominantly on the abluminal side of the vascular endothelium, VEGFR2 available for targeting by circulating ligands is available at the luminal surface.

The international patent applications WO 01/52875 and WO 99/40947 disclose disulphide-cyclic peptides from growth factor proteins modulating the activity of the growth factor, including VEGF-D, for use as angiogenesis inhibiting factors in therapy or in labelled form for diagnosis. US patent application US 2002/114780 discloses the heparin binding domain of VEGF as a target for the delivery of therapeutic agents to tumor tissues.

### The present invention

It has now been found a peptide targeting the heparin binding domain of vascular endothelial growth factor and its receptor vascular endothelial growth factor receptor 2, VEGFR 2. This peptide can be used as a therapeutic agent in a pharmaceutical formulation by inhibiting the angiogenesis in the diseased area/tissue.
Further the peptide can be coupled to a known therapeutic agent that will be carried to the diseased area/tissue by the targeting abilities of the new peptide.
One or more peptide can further be coupled to a chelating agent or a reporter moiety either by direct bonding or via a linker moiety to act as a diagnostic imaging agent or a therapeutic active agent.

### Detailed description of the invention

The invention is described in the patent claims. Specific features of the invention are outlined in the following detailed description and the Examples.

The three letter abbreviations used for the amino acids have the following meaning:
- Arg: - Arginine
- Asp: - Aspartic acid
- Cys: - Cysteine
- Hcy: - Homocysteine
- Gly: - Glycine
- Val: - Valine
- Tyr: - Tyrosine
- Ile: - Isoleucine
- Ser: - Serine
- Leu: - Leucine
- Asn: - Aspargine
- Gln: - Glutamine
- Ala: - Alanine
- Met: - Methionine
- Glu: - Glutamic acid
- His: - Histidine
- Thr: - Threonine
- Phe: - Phenylalanine
- Trp: - Tryptophan
- Ser: - Serine

In a first aspect, the present invention provides a peptide that targets the heparin binding domain of VEGF and its receptor VEGFR 2.

The peptide consists of the amino acid sequence of formula (II) or pharmaceutically acceptable salts thereof

Z¹-X¹-Tyr-X³(-Ala or Ser)-Asp-Gly-X⁷-(Tyr or Phe)-Asp- Z²-Y¹ (II)

wherein
X¹ is an amino acid selected from the group Ser, His, Thr, Ala, Gln, Phe, Gly and Ile
X³ is an amino acid selected from the group Tyr, Ser, Asn, Glu, Asp and Thr
X⁷ is an amino acid selected from the group Thr, Val, Met, Ser, Trp, Tyr, Leu and Ala
Z¹ represent an amino acid residue containing a cysteine or a homocysteine residue, or a residue of formula -(CH₂)n-C(=O)- where n represents a positive integer 1 to 10. wherein the cystein or homocysteine residues or the residues of formula -(CH₂)n-C(=O)- can form a thioether bond or a disphide bond with the cysteine or homocysteine residues of Z², or Z¹ is absent, and
Z² represent cysteine or homocysteine residue which can form a thioether bond or a disulphide bond with the cysteine or homocysteine residues or a residue of formula -(CH₂)n-C(=O)- of Z², or Z² is absent, and
Y¹ represents 1-10 amino acids or is absent.

Further preferred are peptides that consist of an amino acid sequence as follows:
Cys-Ser-Tyr-Tyr-Ser-Asp-Gly-Val-Tyr-Asp-Cys, (SEQ ID NO 1),
Cys-His-Tyr-Ser-Ser-Asp-Gly-Thr-Tyr-Asp-Cys, (SEQ ID NO 2),
Cys-Ala-Tyr-Glu-Ala-Asp-Gly-Trp-Phe-Asp-Cys, (SEQ ID NO 4),
Cys-Thr-Tyr-Ser-Ala-Asp-Gly-Leu-Tyr-Asp-Cys, (SEQ ID NO 8), or
Cys-Gln-Tyr-Thr-Ala-Asp-Gly-Ala-Phe-Asp-Cys. (SEQ ID NO 11).

Viewed from another aspect the invention provides peptide-based compounds as defined by formula (III).

V-L-Z Formula (III)

wherein the vector V is a peptide as defined above, L represents a bond and Z represents an antineoplastic agent represented by cyclophosphamide, chloroambucil, busulphan, methotrexate, cytarabine, fluorouracil, vinblastine, paclitaxel, doxorubicin, daunorubicin, etoposide, teniposide, cisplatin, amsacrine or docetaxel, a reporter moiety or a group that optionally can carry an imaging moiety M.

If Z is represented by an antineoplastic agent, the compound will target an angiogenic site associated with cancer and bring the antineoplastic agent to the diseased area.

The role of the linker L is to couple vector to reporter.

A linker moiety serves to link one vector to one reporter.

The linker moiety L is a simple bond.

Z can be represented by stabilised gas-filled microbubbles. In this aspect of the invention the compounds of formula (III) can be used for targeted ultrasound imaging.

Z can further be represented by a chelating agent of Formula IV: 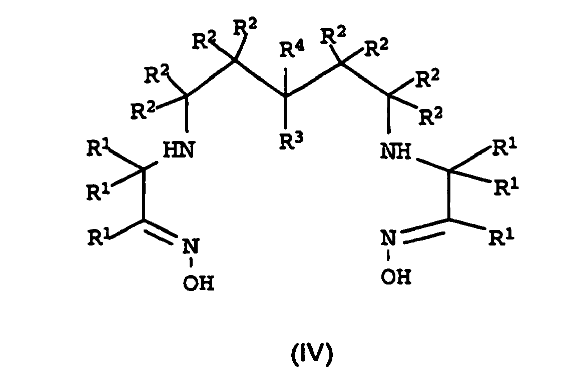 where:
each R¹, R², R³ and R⁴ is independently an R group;
each R group is independently H or C₁₋₁₀ alkyl, C₃₋₁₀ alkylaryl, C₂₋₁₀ alkoxyalkyl, C₁₋₁₀ hydroxyalkyl, C₁₋₁₀ alkylamine, C₁₋₁₀ fluoroalkyl, or 2 or more R groups, together with the atoms to which they are attached form a carbocyclic, heterocyclic, saturated or unsaturated ring, or can represent a chelating agent given by formulas a, b, c and d.

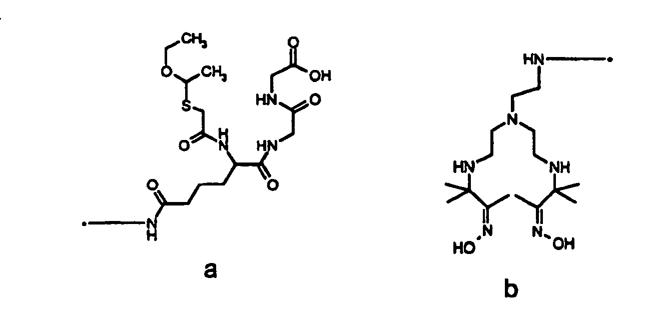

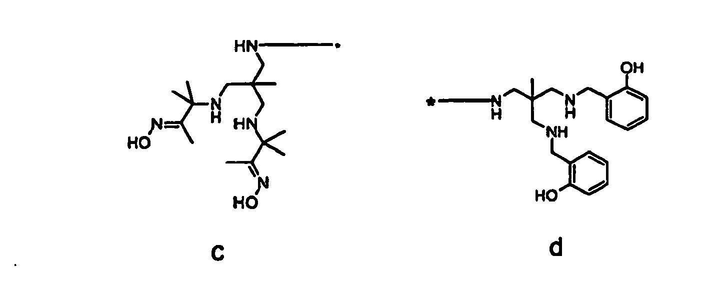

A preferred example of a chelating agent is represented by formula e.

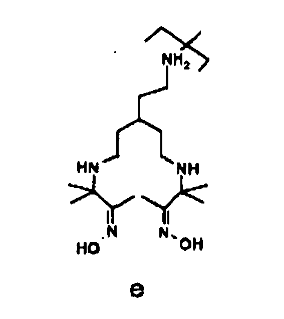

Conjugates comprising chelating agents of Formula (IV) can be radiolabelled to give good radiochemical purity, RCP, at room temperature, under aqueous conditions at near neutral pH. The risk of opening the disulphide bridge of the peptide component at room temperature is less than at an elevated temperature. A further advantage of radiolabelling the conjugates at room temperature is a simplified procedure in a hospital pharmacy.

However the compounds defined in Formula (III) may also comprise chelating agents, Z, as defined in WO 01/77145, Table I, pages 11-15.

In some aspects of the invention, Z comprises a reporter moiety, M, where said reporter moiety comprises a radionuclide. Further definitions of chelating agents are listed in WO 01/77146,Table 1, pages 11-15.

The peptide component of compounds of formula (III) may be in a cyclic configuration, i.e. by a disulphide bond or it may be linear.

The peptide component of the conjugates described herein have preferably no free amino- or carboxy-termini. This introduces into these compounds a significant increase in resistance against enzymatic degradation and as a result they have an increased in vivo stability as compared to many known free peptides.

The reporter moieties (Z) in the contrast agents of the invention may be any moiety capable of detection either directly or indirectly in an in vivo diagnostic imaging procedure.

For MR imaging the reporter will either be a non zero nuclear spin isotope (such as ¹⁹F) or a material having unpaired electron spins and hence paramagnetic, superparamagnetic, ferrimagnetic or ferromagnetic properties; for light imaging the reporter will be a light scatterer (e.g. a coloured or uncoloured particle), a light absorber or a light emitter, for magnetometric imaging the reporter will have detectable magnetic properties; for electrical impedance imaging the reporter will affect electrical impedance; and for scintigraphy, SPECT, PET, and the like, the reporter will be a radionuclide.

Stated generally, the reporter may be (1) a chelatable metal or polyatomic metal-containing ion (i.e. TcO, etc), where the metal is a high atomic number metal (e.g. atomic number greater than 37), a paramagentic species (e.g. a transition metal or lanthanide), or a radioactive isotope, (2) a covalently bound non-metal species which is an unpaired electron site (e.g. an oxygen or carbon in a persistant free radical), a high atomic number non-metal, or a radioisotope, (3) a polyatomic cluster or crystal containing high atomic number atoms, displaying cooperative magnetic behaviour (e.g. superparamagnetism, ferrimagnetism or ferromagnetism) or containing radionuclides.

Examples of particular preferred reporter groups (M) are described in more detail below.

Chelated metal reporters are preferably chosen from the group below; ⁸⁰Y, ^{99m}Tc, ¹¹¹In, ⁴⁷Sc, ⁶⁷Ga, ⁵¹Cr, ^{177m}Sn, ⁶⁷Cu, ¹⁸⁷Tm, ⁹⁷Ru, ¹⁸⁸Re, ¹⁷⁷Lu, ¹⁹⁹Au, ²⁰³Pb and ¹⁴¹Ce.

The metal ions are desirably chelated by chelating agents on the linker moiety. Further examples of suitable chelating agents are disclosed in US-A-4647447, WO89/00557, US-A-5367080, US-A-5364613.

Methods for metallating any chelating agents present are within the level of skill in the art. Metals can be incorporated into a chelant moiety by any one of three general methods: direct incorporation, template synthesis and/or transmetallation. Direct incorporation is preferred.

Thus it is desirable that the metal ion be easily complexed to the chelating agent, for example, by merely exposing or mixing an aqueous solution of the chelating agent-containing moiety with a metal salt in an aqueous solution preferably having a pH in the range of about 4 to about 11. The salt can be any salt, but preferably the salt is a water soluble salt of the metal such as a halogen salt, and more preferably such salts are selected so as not to interfere with the binding of the metal ion with the chelating agent. The chelating agent-containing moiety is preferably in aqueous solution at a pH of between about 5 and about 9, more preferably between pH about 6 to about 8. The chelating agent-containing moiety can be mixed with buffer salts such as citrate, carbonate, acetate, phosphate and borate to produce the optimum pH. Preferably, the buffer salts are selected so as not to Interfere with the subsequent binding of the metal ion to the chelating agent

The following isotopes or isotope pairs can be used for both imaging and therapy without having to change the radio-labelling methodology or chelator. ⁴⁷Sc₂₁; ¹⁴¹Ce₅₈; ¹⁸⁸Re₇₅; ¹⁷⁷Lu₇₁; ¹⁹⁹Au₇₉; ⁴⁷Sc₂₁; ¹³¹I₅₃; ⁶⁷Cu₂₉; ¹³¹I₅₃ and ¹²³I₅₃; ¹⁸⁸Re₇₅ and ^{99m}Tc₄₃; ⁸⁰Y₃₉ and ⁸⁷Y₃₉; ⁴⁷Sc₂₁ and ⁴⁴Sc₂₁; ⁹⁰Y₃₉ and ¹²³I₅₃; ¹⁴⁸Sm₆₂ and ¹⁵³Sm₆₂; and ⁹⁰Y₃₉ and ¹¹¹In₄₉.

Preferred non-metal atomic reporters include radioisotopes such as ¹²³I, ¹³¹I and ¹⁸F as well as non zero nuclear spin atoms such as ¹⁹F, and heavy atoms such as I.

In a further embodiment of this invention, the use of radioisotopes of iodine or fluorine is specifically contemplated. For example, if the peptide or linker is comprised of substituents that can be chemically substituted by iodine or fluorine in a covalent bond forming reaction, such as, for example, substituents containing hydroxyphenyl or p-nitrobenzoyl functionality, such substituents can be labeled by methods well known in the art with a radioisotope of iodine or fluorine respectively. These species can be used in therapeutic and diagnostic imaging applications. While, at the same time, a metal attached to a chelating agent on the same peptide-linker can also be used in either therapeutic or diagnostic imaging applications.

The compounds of formula (III) may be therapeutically effective in the treatment of disease states as well as detectable in in vivo imaging. Thus for example the vector on the reporter moieites may have therapeutic efficacy, e.g. by virtue of the radiotherapeutic effect of a radionuclide reporter of the vector moiety.

The present invention also provides a pharmaceutical composition comprising an effective amount (e.g. an amount effective for enhancing image contrast in in vivo imaging) of a compound of general formula (III) or a salt thereof, together with one or more pharmaceutically acceptable adjuvants, excipients or diluents.

The invention further provides a pharmaceutical composition for treatment of a disease comprising an effective amount of a compound of general formula (III), or a salt thereof, together with one or more pharmaceutically acceptable adjuvants, excipients or diluents.

Use of the new peptide and the compounds of formula (III) in the manufacture of therapeutic compositions (medicament) and in methods of therapeutic or prophylactic treatment, preferably treatment of cancer, of the human or animal body are thus considered to represent further aspects of the invention.

Viewed from a further aspect the invention provides the use of a compound of formula (III) for the manufacture of a contrast medium for use in a method of diagnosis involving administration of said contrast medium to a human or animal body and generation of an image of at least part of said body.

The compounds of the invention may be used in therapeutically effective treatments as well as for imaging purposes. Further these compounds may be used for drug delivery purposes.

### General procedures

The abbreviations used have the following meanings:
- Fmoc: - 9-fluorenylmethoxycarbonyl
- Acm: - acetamidomethyl
- TFA: - trifluoroacetic acid
- ACN: - acetonitril
- RP-HPLC: - reversed phase high pressure liquid chromatography
- DMF: - dimethylformamide
- NMM: - N-methylmorpholine
- DCM: - dichloromethane
- NMP: - N-methylpyrrolidone
- HBTU: -2-(1H-benzotriazol-1-yl)-1,1,3,3-tetramethyluronium-hexafluorophosphate
- HOBt: - 1-hydroxy-benzotriazole
- DIEA: - diisopropylethylamine
- DTT: - dithiotreitol

The peptides of the present invention can be synthesised using all the known methods of chemical synthesis but particularly useful is the solid-phase methodology of Merrifield employing an automated peptide synthesiser (J. Am. Chem. Soc., 85: 2149 (1964)). Typically, the desired sequences are assembled by solid-phase peptide synthesis. Standard procedures for the synthesis strategy employed for the examples of this invention are described in E. Atherton & R.C. Sheppard, "Solid phase peptide synthesis: a practical approach, 1989, IRL Press, Oxford.

For example, a synthesis resin with an acid-labile linker group, to which the desired protected C-terminal amino acid residue is attached by amide bond formation, is used. In the following examples 1 and 2, a so-called Rink amide AM resin with a (dimethoxyphenyl-aminomethyl)-phenoxy-derived linker was applied (Rink, H. Tetrahedron Lett. (1987), 30, 3787). Moreover, in example 3 an even more acid labile resin was used, a so-called XAL-MBHA resin with a xanthenyl-derived linker (Han, Y.; Bontems, S. L.; Hegyes, P.; Munson, M. C.; Minor, C. A.; Kates, S. A.; Albericio, F.; Barany, G., J. Org. Chem. (1996), 61, 6326-6339).

The N^{α}-amino-protecting group is then removed and the second amino acid in the sequence is coupled using suitable condensation reagents. N^{α}-amino-deprotection and coupling cycles are then repeated in alternating steps until the sequence of interest is assembled. Amino acids with a temporary N^{α}-amino protecting group and permanent protecting groups for the functional side chains are employed. Generally, all reactive groups present (for example amino, hydroxyl, thiol and carboxyl groups) will be protected during peptide synthesis.
A wide range of protecting groups for amino acids is known (see, e.g., Greene, T.W. & Wuts, P.G.M. (1991) Protective groups in organic synthesis, John Wiley & Sons, New York). Thus, examples of amino protecting groups which may be employed include 9-fluorenylmethoxycarbonyl (Fmoc), benzyloxycarbonyl, t-butyloxycarbonyl, ect. When the peptide is built up from the C-terminal end, the N°-amino-protecting group will need to be removed selectively prior to the next coupling step. One particularly useful group for such temporary amine protection is the Fmoc group which can be removed selectively by treatment with piperidine in an organic solvent. Carboxyl protecting groups which may be employed include for example t-butyl, benzyl, trityl, etc. The thiol protecting group used can be semi-permanent for example the *p*-methoxytrityl group or permanent including the trityl and acetamidomethyl groups. It will, be appreciated that a wide range of other such groups are known in the art.

Finally the peptide is cleaved from the synthesis resin and the permanent side-chain protecting groups are removed, usually simultaneously as in examples 1 and 2 below. Alternatively, a synthesis resin is used which allows detachment of the peptide under mild conditions where the side-chain protecting groups are stable, affording protected peptides, as in example 3 below.

The invention is further illustrated by the following none limiting examples.

### Examples

### Example 1

### cyclo-[CH₂CO-Ser-Tyr-Tyr-Ser-Asp-Gly-Val-Tyr-Asp-Cys]-Gly-Cys-NH₂

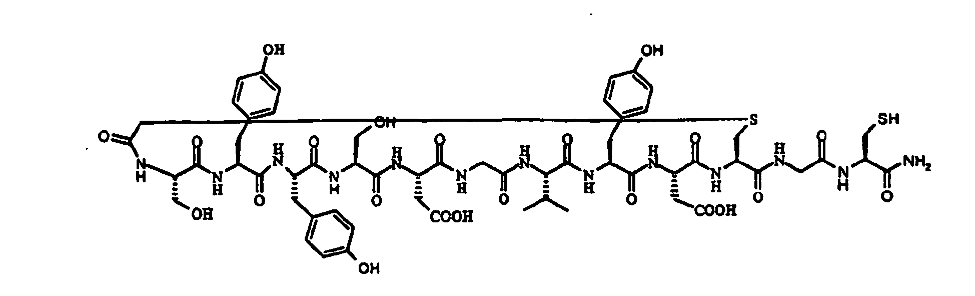

The peptidyl resin corresponding to the above sequence was assembled on a Rink Amide AM resin (0.74 mmol/g; from NovaBiochem) using an Applied Biosystems (Perkin Elmer) model 433A peptide synthesizer. Fmoc deprotection was achieved with conductivity monitoring using 20% piperidine in N-methylpyrrolidone (NMP). The washing solvent was NMP. The residues (from the carboxyl terminus) were assembled on a 0.25 mmol scale using single couplings with a 4-fold molar excess of Fmoc-amino acids (1 mmol cartridges) and 2-(1H-benzotriazol-1-yl)-1,1.3.3-tetramethyluronium-hexafluorophosphate (HBTU)/1-hydroxy-benzotriazole (HOBt)/diisopropylethylamine (DIEA) in NMP using 2.5 hours coupling cycles. The amino acid-side chain protecting groups used were *tert*-butyl for Asp, Ser and Tyr, trityl for Cys¹⁰ and acetamidomethyl (Acm) for Cys¹². The Gly residue coupled in position 6 was used with an additional N°-protecting group (2-Fmoc-oxy-4-methoxybenzyl) in order to suppress aspartimide formation (Packman, L.C. 1995, Tetrahedron Lett. 36, p.7523-7526).

The assembled peptidyl resin was then transferred to a manual nitrogen bubbler apparatus (Wellings, D.A., Atherton, E. (1997) in Methods in Enzymology (Fields, G. ed), 289, p. 53-54, Academic Press, New York). The N-terminus was Fmoc-deprotected and then bromoacetylated using a 10-fold molar excess of bromoacetyl bromide and N-methylmorpholine (NMM) in dimethylformamide (DMF) for 1hour. The completed peptidyl resin was washed with DMF and dichloromethane (DCM) and dried *in vacuo.*

Simultaneous cleavage of the peptide from the resin and removal of the side-chain protecting groups (except the acetamidomethyl group) from the peptide, was effected by treating an aliquot of the peptidyl resin (0.1 mmol) with trifluoroacetic acid (TFA) containing 2.5% triisopropylsilane and 2.5% water for 2 hours. The resin residue was filtered off and washed with small quantities of neat TFA. The combined filtrate and washings were concentrated by rotary evaporation and then triturated with diethyl ether to obtain the crude peptide. The precipitate was isolated by centrifugation, washed with ether and then lyophilized from 50% acetonitrile (ACN)-0.1 % aq TFA giving a crude product yield of 150 mg.

The linear crude peptide was cyclized by thioetherbridge formation, effected by stirring the peptide in 300 ml 50% ACN-water at pH 8 (adjusted by liquid ammonia) for 30 min at RT. The cyclized product was isolated by lyophilization. The Acm-protecting group was then removed by treating the peptide with a 5-fold molar excess of mercury(II)-acetate at pH 4-4.5 (adjusted with acetic acid) in 25% ACN-water. After stirring for 1 h at RT dithiotreitol (DTT) was added as a solid to the mixture in a 4-fold molar excess with respect to mercury(II)-acetate. The reaction was stirred for 2.5 h at RT and the precipitate that formed was removed by centrifugation. The supernatant was lyophilized to give the fully deprotected cyclized crude peptide. An aliquot (0.074 mmol) of the crude product was dissolved in 10% ACN-0.1% aq. TFA (50 ml) filtered and purified by preparative RP-HPLC. The column (Phenomenex Luna C18 10µ, 250 x 50 mm) was eluted at 50 ml/ min with a gradient of 10 to 25% ACN in 0.1% aq TFA over 60 min. The desired peak fractions were pooled and lyophilized to afford 21 mg of pure peptide. Analytical RP-HPLC: t_{R} = 17.2 min, purity 99% (Phenomenex Luna 5µ, 4.6 x 250 mm, 0-40 % ACN in 0.1% aq TFA over 20 min at 1 ml/min, λ=215 nm). Electrospray MS: [M+H]+ of product expected at 1370.5 m/z, found at 1370.3 m/z.

### Example 2

### Disulfide-Ala-Glu-Gly-Glu-Phe-Cys-Ser-Tyr-Tyr-Ser-Asp-Gly-Val-Tyr-Asp-Cys-Gly-Cys-NH₂

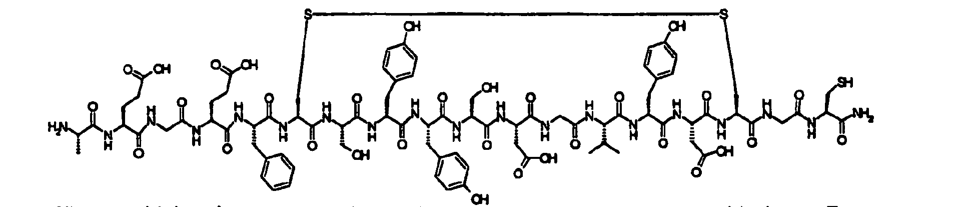

The peptidyl resin corresponding to the above sequence was assembled on a Fmoc-XAL-MBHA resin (0.47 mmol/g; from Bachem) in a similar fashion to the corresponding peptidyl resin of Example 1. Selective removal of the peptide from the resin while maintaining the side-chain protecting groups, was carried out on a 0.085 mmol scale using the procedure described in Example 1.

The reduced protected peptide was oxidized by re-dissolving the crude peptide in DMF (25 ml) and adding the solution to 65% acetonitrile (ACN)-H₂O (750 ml). It was difficult to obtain a homogeneous solution so more ACN (250 ml), DMF (100ml) and DMSO (200 ml) was added in order to obtain a close to homogeneous solution. The pH was adjusted to -7.9 with dilute liq. NH₃ and the oxidation was let proceed for 48 h. At this point the solution was concentrated down by rotary evaporation.

### Example 3

### Cys1-11; Cys-Ser-Tyr-Tyr-Ser-Asp-Gly-Val-Tyr-Asp-Cys-Gly-Cys-NH₂

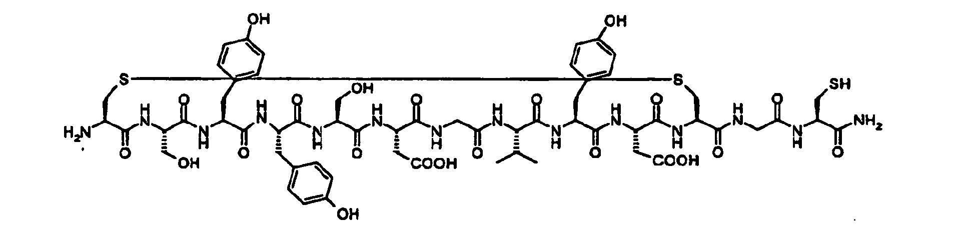

The peptidyl resin corresponding to the above sequence was assembled on a Fmoc-XAL-MBHA resin (0.47 mmol/g; from Bachem) in a similar fashion to the corresponding, peptidyl resin of Example 1. The side-chain protecting groups for the Cys residues used here were *p*-methoxytrityl for Cys^{1,11} and trityl for Cys¹³.

An aliquot of the assembled peptidyl resin (0.035 mmol) was transferred to a manual nitrogen bubbler and treated with DCM containing 5% TIS and 1% TFA for 5 x 5 min in order to liberated the fully side-chain protected peptide from the resin. Care was taken to avoid evaporation of DCM and thus up-concentration of TFA. The filtrates were neutralized by collection in a flask containing pyridine (2 equivalents pr equivalent TFA, 1 ml) and MeOH (1 ml /10 ml cleavage solution, 5 ml). The combined filtrates were concentrated by rotary evaporation until precipitation occurred and the precipitate was isolated by centrifugation.

Cyclization of the protected peptide by disulfide formation was carried out by adding a solution of the peptide in DMF (10 ml) to 60% ACN-water (250 ml), giving a concentration of <0.25 mg/ml. The pH was adjusted to -8.6 with dilute liq. NH₃ and the oxidation was let proceed for 40 h. At this point the solution was concentrated down and the product was isolated by centrifugation.

An aliquot (-18 mg)) of the crude product was dissolved in 1 ml DMF and 3 ml 65% ACN-0.1% aq TFA filtered and purified by preparative RP-HPLC. The column (Vydac 218 TP1022, 22 x 250 mm) was eluted at 10 ml/ min with a gradient of 65 to 100% ACN in 0.1 % aq TFA over 40 min. The desired peak fractions were pooled and lyophilized. Analytical RP-HPLC: t_{R} = 10.3 min, purity 95% (Phenomenex Luna 3µ, 2.0 x 50 mm, 50-100 % ACN in 0.1% aq TFA over 10 min at 0.4 ml/min, λ=215 nm). MALDI-MS: [M+Na]⁺ of product expected at 2225.8 m/z, found at 2230.9 m/z.

Final deprotection of the peptide was effected by acidolytic treatment for 1 hr as described in Example 1. Yield: 1.3 mg. Analytical RP-HPLC: t_{R} = 5.97 min, purity 65% (Phenomenex Luna 3µ, 4.6 x 50 mm, 0-40 % ACN in 0.1% aq TFA over 10 min at 2 ml/min, λ=215 nm). ESI-MS: [M+H]⁺ of product expected at 1431.5 m/z, found at 1431.4 m/z. ESI-MS/MS: [M+H]⁺ of the *des*-Gly-Cys product expected at 1254.4 m/z, found at 1254.3 m/z. The peptide product was used as such without further purification.

### SEQUENCE LISTING<110> Amersham Health AS<120>

Contrast Agents<130> PN0273<160> 14 <170> PatentIn version 3.1<210> 1<211> 11<212> PRT<213> Artificial sequence<220><223> syntetic peptide
<400> 1
<210> 2<211> 11<212> PRT<213> Artificial sequence<220><223> Syntetic peptide
   <400> 2
   Cys His Tyr Ser Ser Asp Gly Thr Tyr Asp Cys
   1               5                   10
<210> 3<211> 11<212> PRT<213> Artificial sequence<220><223> Syntetic peptide
   <400> 3
   Cys Thr Tyr Asn Gly Asp Gly Ser Phe Asp Cys
   1               5                   10
<210> 4<211> 11<212> PRT<213> Artificial sequence<220><223> Syntetic peptide
   <400> 4
   Cys Ala Tyr Glu Ala Asp Gly Trp Phe Asp Cys
   1               5                   10
<210> 5<211> 11<212> PRT<213> Artificial sequence<220><223> syntetic peptide
   <400> 5
   Cys Ser Tyr Ser Ala Asp Gly Thr Leu Asp Cys
   1               5                   10
<210> 6<211> 11<212> PRT<213> Artificial sequence<220><223> Syntetic peptide
   <400> 6
   Cys Gln Tyr Asp Ser Ser Gly Met Tyr Asp Cys
   1               5                   10
<210> 7<211> 11<212> PRT<213> Artificial sequence<220><223> Syntetic peptide
   <400> 7
   Cys Phe Phe Asp Ser Ser Gly Tyr Phe Asp Cys
   1 5 10
<210> 8<211> 11<212> PRT<213> Artificial sequence<220><223> syntetic peptide
   <400> 8
   Cys Thr Tyr Ser Ala Asp Gly Leu Tyr Asp Cys
   1 5 10
<210> 9<211> 11<212> PRT<213> Artificial sequence<220><223> syntetic peptide
   <400> 9
   Cys His Phe Asp Gly Asp Gly Ser Tyr Asp Cys
   1 5 10
<210> 10<211> 11<212> PRT<213> Artificial sequence<220><223> Syntetic peptide
   <400> 10
   Cys Thr Tyr Glu Pro Ser Gly Met Tyr Asp Cys
   1 5 10
<210> 11<211> 11<212> PRT<213> Artificial sequence<220><223> Syntetic peptide
   <400> 11
   Cys Gln Tyr Thr Ala Asp Gly Ala Phe Asp Cys
   1 5 10
<210> 12<211> 11<212> PRT<213> Artificial sequence<220><223> syntetic peptide
   <400> 12
   Cys Ile Tyr Glu Ser Asp Gly Met Phe Ser Cys
   1 5 10
<210> 13<211> 11<212> PRT<213> Artificial sequence<220><223> syntetic peptide
   <400> 13
   Cys Gly Arg Ser Asp Gly Thr Trp Tyr Glu Cys
   1 5 10
<210> 14<211> 11<212> PRT<213> Artificial sequence<220><223> Syntetic peptide
   <400> 14
   Cys Ser Tyr Tyr Ala Asp Gly Met Tyr Ser Cys
   1 5 10

## Claims

1. A peptide of the amino acid sequence of formula (II) or pharmaceutically acceptable salts thereof
Z¹-X¹-Tyr-X³(-Ala or Ser)-Asp-Gly-X⁷-(Tyr or Phe)-Asp- Z²-Y¹ (II)
wherein
X¹ is an amino acid selected from the group Ser, His, Thr, Ala, Gln, Phe, Gly and Ile
X³ is an amino acid selected from the group Tyr, Ser, Asn, Glu, Asp and Thr
X⁷ is an amino acid selected from the group Thr, Val, Met, Ser, Trp, Tyr, Leu and Ala
Z¹ represent an amino acid residue containing a cysteine or a homocysteine residue, or a residue of formula -(CH₂)n -C(=O)- where n represents a positive integer 1 to 10, wherein the cysteine or homocysteine residues or the residues of formula -(CH₂)ₙ -C(=O)- can form a thioether bond or a disulphide bond with the cysteine or homocysteine residues of Z², or Z¹ is absent, and
Z² represent a cysteine or a homocysteine residue which can form form a thioether bond or a disulphide bond with the cysteine or homocysteine residues or a residue of formula -(CH₂)n -C(=O)- of Z², or Z² is absent, and
Y¹ represents 1-10 amino acids or is absent.

2. A peptide according to claim 1 of the amino acid sequence
Cys-Ser-Tyr-Tyr-Ser-Asp-Gly-Val-Tyr-Asp-Cys, (SEQ ID NO 1),
Cys-His-Tyr-Ser-Ser-Asp-Gly-Thr-Tyr-Asp-Cys, (SEQ ID NO 2),
Lys-Ala-Tyr-Glu-Ala-Asp-Gly-Trp-Phe-Asp-Cys, (SEQ ID NO 4),
Cys-Thr-Tyr-Ser-Ala-Asp-Gly-Leu-Tyr-Asp-Cys, (SEQ ID NO 8),
Cys-Gln-Tyr-Thr-Ala-Asp-Gly-Ala-Phe-Asp-Cys, (SEQ ID NO 11),

3. A peptide of claim 1 of one of the formulas

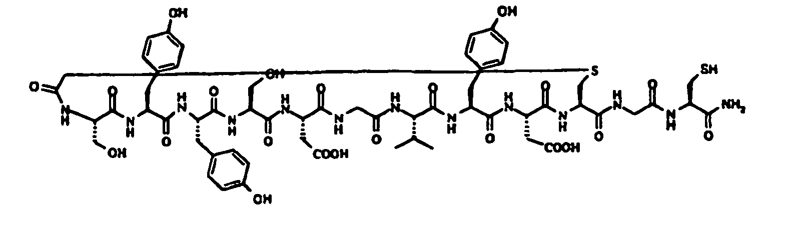

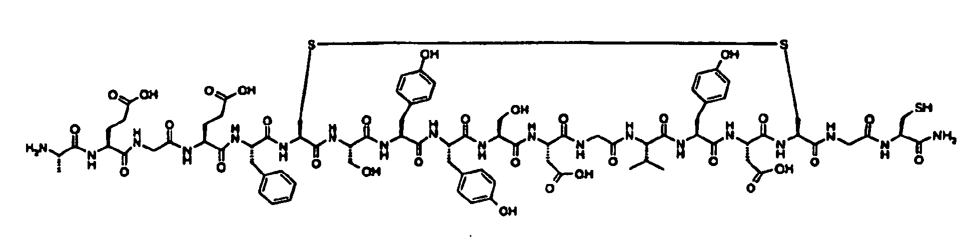

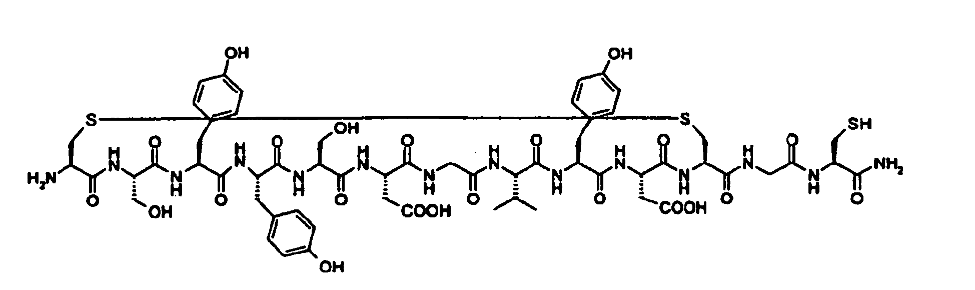

4. A targetable diagnostic and/ or therapeutically active agent of formula (III)
V-L-Z Formula (III)
wherein the vector V is a peptide according to claim 1- 2
L represents a bond and
Z represents an antineoplastic agent of cyclophosphamide, chloroambucil, busulphan, methotrexate, cytarabine, fluorouracil, vinblastine, paclitaxel, doxorubicin, daunorubicin, etoposide, teniposide, cisplatin, amsacrine or docetaxel, a reporter moiety or a group that optionally can carry an imaging moiety M.

5. An agent as claimed in claim 4 where Z is a chelating agent of Formula IV 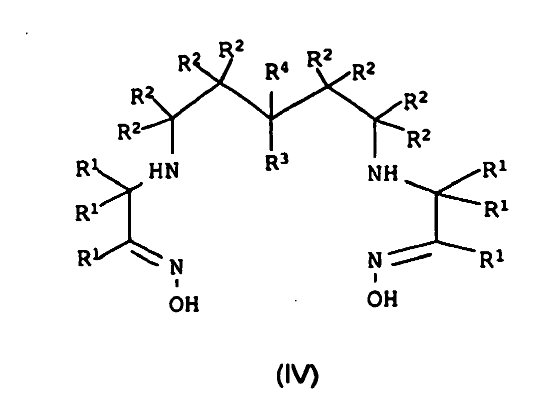 where:
each R¹, R², R³ and R⁴ is independently an R group;
each R group is independently H or C₁₋₁₀ alkyl, C₃₋₁₀ alkylaryl, C₂₋₁₀ alkoxyalkyl, C₁₋₁₀ hydroxyalkyl, C₁₋₁₀ alkylamine, C₁₋₁₀ fluoroalkyl, or 2 or more R groups, together with the atoms to which they are attached form a carbocyclic, heterocyclic, saturated or unsaturated ring.

6. An agent as claimed in any of the previous claims 4 to 5 wherein Z comprises a reporter moiety M wherein the reporter moiety M comprises metal radionuclides, paramagnetic metal ions, fluorescent metal ions, heavy metal ions or cluster ions.

7. An agent as claimed in claim 6 wherein the reporter moiety M comprises ⁹⁰Y, ^{99m}Tc, ¹¹¹In, ⁴⁷Sc, ⁶⁷Ga, ⁵¹Cr, ^{117m}Sn, ⁸⁷Cu, ¹⁸⁷Tm, ⁹⁷Ru, ¹⁸⁸Re, ¹⁷⁷Lu, ⁹⁹Au, ²⁰³Pb, ¹⁴¹Ce or ¹⁸F.

8. A pharmaceutical composition comprising an effective amount of a compound of general Formula (III) or a salt thereof, together with one or more pharmaceutically acceptable adjuvants, excipients or diluents for use in enhancing image contrast in *in vivo* imaging or for treatment of a disease.

## Patentansprüche

1. Peptid der Aminosäuresequenz der Formel (II) oder pharamazeutisch annehmbare Salze davon.
Z¹-X¹-Tyr-X³(-Ala or Ser)-Asp-Gly-X⁷-(Tyr oder Phe)-Asp-Z²-Y¹ (II)
wobei
X¹ steht für eine Aminosäure, ausgewählt aus der Gruppe Ser, His, Thr, Ala, Gln, Phe, Gly und Ile,
X³ steht für eine Aminosäure, ausgewählt aus der Gruppe Tyr, Ser, Asn, Glu, Asp und Thr
X^{Y} steht für eine Aminosäure, ausgewählt aus der Gruppe Thr, Val, Met, Ser, Trp, Tyr, Leu und Ala,
Z¹ einen Aminosäurerest repräsentiert, der enthält einen Cystein- oder einen Homocysteinrest, oder einen Rest der Formel -(CH₂)n-C(=O)- wobei n eine positive ganze Zahl von 1-10 repräsentiert, wobei die Cystein- oder Homocysteinreste oder die Reste der Formel -(CH₂)n-C(=O)- eine Thioetherbindung oder eine Disulphidbindung bilden können mit den Cystein- oder Homocysteinresten von Z², oder Z¹ nicht vorhanden ist, und
Z² einen Cystein- oder einen Homocysteinrest repräsentiert, der eine Thioetherbindung oder eine Disulphidbindung mit den Cystein- oder Homocystein-Resten oder einem Rest der Formel -(CH₂)n-C(=O)- von Z² bilden kann, oder Z² nicht vorhanden ist, und
Y¹ 1-10 Aminosäuren repräsentiert oder nicht vorhanden ist.

2. Peptid nach Anspruch 1 der Aminosäuresequenz
Cys-Ser-Tyr-Tyr-Ser-Asp-Gly-Val-Tyr-Asp-Cys, (SEQ ID Nr.1)
Cys-His-Tyr-Ser-Ser-Asp-Gly-Thr-Tyr-Asp-Cys, (SEQ ID Nr. 2)
Lys-Ala-Tyr-Gly-Ala-Asp-Gly-Trp-Phe-Asp-Cys, (SEQ ID Nr. 4)
Cys-Thr-Tyr-Ser-Ala-Asp-Gly-Leu-Tyr-Asp-Cys, (SEQ ID Nr. 8)
Cys-Gln-Tyr-Thr-Ala-Asp-Gly-Ala-Phe-Asp-Cys, (SEQ ID Nr. 11)

3. Peptid nach Anspruch 1 nach einer der Formeln

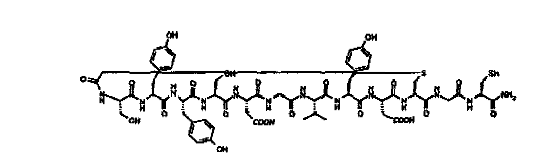

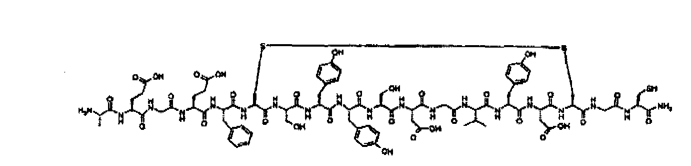

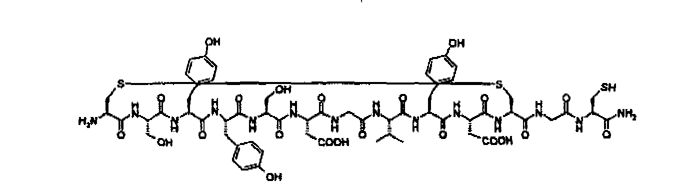

4. Zielausrichtbares diagnostisches und/oder therapeutisch aktives Mittel der Formel (III)
V-L-Z Formel (III)
wobei der Vektor V für ein Peptid steht nach den Ansprüchen 1-2,
L eine Bindung repräsentiert, und
Z repräsentiert ein antineoplastisches Mittel von Cycloposphamid, Chloroambucil,
Busulphan, Methotrexat, Cytarabin, Fluorouracil, Vinblastin, Paclitaxel, Doxorubicin, Daunorubicin, Etoposide, Teniposide, Cisplatin, Amsacrin oder Docetaxel, einer Reportereinheit oder einer Gruppe, die ggf. eine Bildgebungseinheit M tragen kann.

5. Mittel nach Anspruch 4, wobei Z für ein chelatierendes Mittel der Formel (IV) steht. 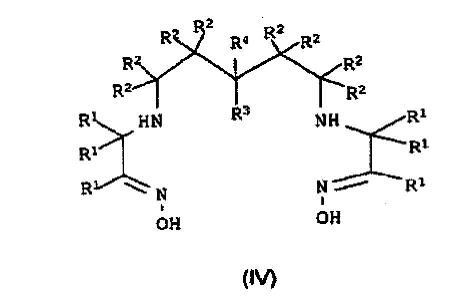 wobei:
jedes R¹, R², R³ und R⁴ unabhängig steht für eine R-Gruppe;
jede R-Gruppe unabhängig steht für H oder C₁₋₁₀Alkyl, C₃₋₁₀-Alkylaryl, C₂₋₁₀-Alkoxyalkyl, C₁₋₁₀-Hydroxyalkyl, C₁₋₁₀-Alkylamin, C₁₋₁₀-Fluoroalkyl, oder 2 oder mehr R-Gruppen, zusammen mit den Atomen, an die sie gebunden sind, einen carbocyclischen, heterocyclischen, gesättigten oder ungesättigten Ring bilden.

6. Mittel nach einem der vorstehenden Ansprüche 4 bis 5, wobei Z umfasst eine Reportereinheit M, wobei die Reportereinheit M umfasst Metallradionuklide, paramagnetische Metallionen, fluoreszierende Metallionen, Schwermetallionen oder Clusterionen.

7. Mittel nach Anspruch 6, wobei die Reportereinheit M umfasst
⁹⁰Y, ^{99m}Tc, ¹¹¹In, ⁴⁷Sc, ⁶⁷Ga, ⁵¹Cr, ^{177m}Sn, ⁶⁷Cu, ¹⁸⁷Tm, ⁹⁷Ru, ¹⁸⁸Re, ¹⁷⁷Lu, ¹⁹⁹Au, ²⁰³Pb, ¹⁴¹Ce oder ¹⁸F.

8. Pharmazeutische Zusammensetzung, umfassend eine wirksame Menge einer Verbindung der allgemeinen Formel (III) oder eines Salzes davon, zusammen mit einem oder mehreren pharmazeutisch annehmbaren Hilfsmitteln, Hilfsstoffen oder Verdünnungsmitteln zur Verwendung beim Verstärken des Bildkontrastes in der in-vivo-Bildgebung oder für die Behandlung einer Krankheit.

## Revendications

1. Peptide de la séquence d'acides aminés répondant à la formule (II) ou sels pharmaceutiquement acceptables de celui-ci
Z¹ -X¹ -Tyr-X³(-Ala ou Ser)-Asp-Gly-X⁷-(Tyr ou Phe)-Asp-Z² -Y¹ (II)
dans laquelle
X¹ est un acide aminé choisi dans le groupe constitué par Ser, His, Thr, Ala, Gln, Phe, Gly et Ile
X³ est un acide aminé choisi dans le groupe constitué par Tyr, Ser, Asn, Glu, Asp et Thr
X⁷ est un acide aminé choisi dans le groupe constitué par Thr, Val, Met, Ser, Trp, Tyr, Leu et Ala
Z¹ représente un reste d'acide aminé contenant un reste de cystéine ou d'homocystéine, ou un reste répondant à la formule -(CH₂)n -C(=O)- dans laquelle n représente un nombre entier positif allant de 1 à 10, les restes cystéine ou homocystéine ou les restes répondant à la formule -(CH₂)n - C(=O)- pouvant former une liaison thioéther ou une liaison disulfure avec les restes de cystéine ou d'homocystéine de Z², ou bien Z¹ est absent, et
Z² représente un reste de cystéine ou d'homocystéine capable de former une liaison thioéther ou une liaison disulfure avec les restes de cystéine ou d'homocystéine ou un reste répondant à la formule -(CH₂)n -C(=O)- de Z², ou Z² est absent, et
Y¹ représente de 1 à 10 acides aminés ou est absent

2. Peptide selon la revendication 1 de la séquence d'acides aminés
Cys-Ser-Tyr-Tyr-Ser-Asp-Gly-Val-Tyr-Asp-Cys, (SEQ ID NO 1),
Cys-His-Tyr-Ser-Ser-Asp-Gly-Thr-Tyr-Asp-Cys, (SEQ ID NO 2),
Cys-Ala-Tyr-Glu-Ala-Asp-Gly-Trp-Phe-Asp-Cys, (SEQ ID NO 4),
Cys-Thr-Tyr-Ser-Ala-Asp-Gly-Leu-Tyr-Asp-Cys, (SEQ ID NO 8),
Cys-Gln-Tyr-Thr-Ala-Asp-Gly-Ala-Phe-Asp-Cys, (SEQ ID NO 11).

3. Peptide selon la revendication 1 répondant à l'une des formules

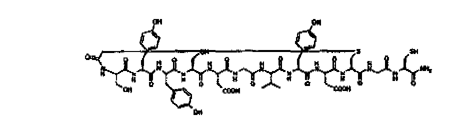

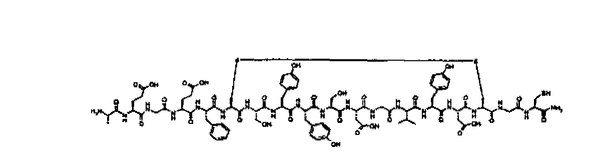

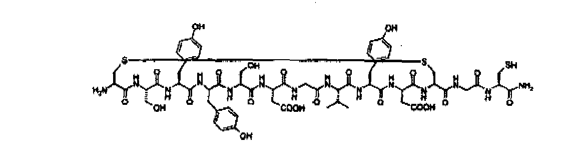

4. Agent de diagnostic et/ou thérapeutiquement actif pouvant être ciblé répondant à la formule (III)
V-L-Z Formule (III)
dans laquelle le vecteur V est un peptide selon les revendications 1 à 2
L représente une liaison et
Z représente un agent antinéoplastique de cyclophosphamide, chlorambucil, busulfan, méthotrexate, cytarabine, fluorouracil, vinblastine, paclitaxel, doxorubicine, daunorubicine, étoposide, teniposide, cisplatine, amsacrine, ou docétaxel, un fragment rapporteur ou un groupe pouvant éventuellement porter un fragment d'imagerie M.

5. Agent selon la revendication 4 dans lequel Z est un agent chélatant répondant à la formule IV 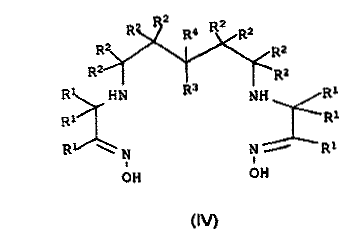 dans laquelle :
chaque R¹, R², R³ et R⁴ représente indépendamment un groupe R ;
chaque groupe R représente indépendamment un atome d'hydrogène ou un groupe alkyle en C₁-C₁₀, alkylaryle en C₃-C₁₀, alcoxyalkyle en C₂-C₁₀, hydroxyalkyle en C₁-C₁₀, alkylamine en C₁-C₁₀, fluoroalkyle en C₁-C₁₀, ou bien 2 groupes R, ou plus, conjointement avec les atomes auxquels ils sont rattachés, forment un cycle carbocyclique, hétérocyclique, saturé ou insaturé.

6. Agent selon l'une quelconque des revendications précédentes 4 à 5 dans lequel Z comprend un fragment rapporteur M, lequel fragment rapporteur M comprend des radionucléides métalliques, des ions métalliques paramagnétiques, des ions métalliques fluorescents, des ions métalliques lourds ou des ions d'agrégats.

7. Agent selon la revendication 6 dans lequel le fragment rapporteur M comprend ⁹⁰Y, ^{99m}Tc, ¹¹¹In, ⁴⁷Sc, ⁶⁷Ga, ⁵¹Cr, ^{177m}Sn, ⁶⁷Cu, ¹⁶⁷Tm, ⁹⁷Ru, ¹⁸⁸Re, ¹⁷⁷Lu, ¹⁹⁹Au, ²⁰³Pb, ¹⁴¹Ce, ou ¹⁸F.

8. Composition pharmaceutique comprenant une quantité efficace d'un composé de formule générale (III) ou d'un sel de celui-ci, ainsi qu'un ou plusieurs adjuvants, excipients ou diluants pharmaceutiquement acceptables, destinée à permettre le perfectionnement du contraste d'image dans l'imagerie *in vivo* ou à être mise en oeuvre dans le traitement d'une maladie.
